# EUROPEAN PATENT SPECIFICATION

(11) **EP 3 784 393 B1**
(45) Date of publication and mention of the grant of the patent: **10.07.2024**
(21) Application number: 19725393.3
(22) Date of filing: 25.04.2019
(51) Int. Cl.: B01L 3/00, C12Q 1/686, B01L 7/00

(54) **METHODS**
VERFAHREN
PROCÉDÉS

(30) Priority: 25.04.2018 GB 201806762
(43) Date of publication of application: 03.03.2021
(73) Proprietor: BG Research Ltd, Kimbolton, Cambridgeshire PE28 0NJ (GB)
(72) Inventor: NAZARETH, Nelson, Cambridgeshire PE28 0NJ (GB); EDGE, David, Cambridgeshire PE28 0NJ (GB); TYLER, Adam, Cambridgeshire PE28 0NJ (GB); SADLER, Matthew, Cambridgeshire PE28 0NJ (GB)
(74) Representative: Potter Clarkson
(86) International application number: PCT/GB2019/051156
(87) International publication number: WO 2019/207308

(56) References cited:
- WO-A2-2017/055791
- US-A1- 2010 203 595
- US-A1- 2014 339 191
- US-A1- 2015 290 640
- US-A1- 2016 354 775
- US-A1- 2017 239 653
- US-A1- 2017 268 043
- US-B1- 6 783 993

## Description

### Field of Invention

The present invention relates to the field of pathogen detection.

### Background

Typically, molecular methods for screening for the presence of high containment level pathogens such as Ebola requires additional levels of biosecurity in order to protect the operator performing the test. The first step of screening for these viral haemorrhagic fevers is the taking of a venous draw of blood from the patient. The individual taking the sample will typically be wearing full personal protection which includes multiple pairs of gloves, a protective suit and face mask. Subsequent to taking the blood sample the outside of the collection vessel is sterilised by dunking in bleach and then a virucide, for example guanidium isothiocyanate, to render the virus non-infectious before a nucleic acid extraction takes place. These known methods comprise multi-step procedures and hence require trained users and access to a laboratory.

The typical volumes used in a venous draw for such testing are 3-6ml and as such the risk of exposure to the pathogen is high when taking into account that multiple liquid transfers and the removal of caps must take place. If a smaller volume, such as a smaller volume of blood could be directly processed then this risk of exposure could be minimised and if the reaction vessel where the direct molecular test took place could be designed such that it possessed multiple biosecurity features then the risk of pathogen release could be greatly minimised.

If a smaller volume of sample, for example of blood is to be processed, in order to maximise operator safety, then there is increased onus placed on the requirement for sensitivity. The WHO R&D blueprint (https://www.who.int/blueprint/en/) defines a level of 3,000 virions/ml of whole blood as being a suitable level of detection for low cost diagnostics for the developing world. This would make possible the detection of a wide range of diseases including HIV and Hepatitis C, and including the viral haemorrhagic fevers described above (Ebola, Lassa, Marburg, Rift Valley fever, Crimean Congo fever, Nipah, Yellow fever, Dengue and others). The figure of 3,000 virions/ml would then provide the requirement for lower limit of detection and give guidance on the volume of blood required to be directly added into the reaction, since 3,000/ml is 3 viral targets per microlitre.

Improvements to current methods and equipment are required to allow safer detection of pathogens, particularly in instances where access to safety equipment is limited, such as in certain third-world countries, which coincide with a high incidence of some of the most deadly pathogens.

Blood is an easily accessible and commonly used source of potential pathogens that is routinely used in diagnosis.

Pathogens are routinely detected via PCR based diagnostics, including rtPCR. However, PCR is inhibited by the presence of whole blood, both in terms of the enzymatic amplification process and the optics used to detect the presence of the PCR product in some forms of PCR, such as rtPCR. Whole blood contains inhibitors such as haem, iron, immunoglobulins and others that inhibit the polymerase enzymes required to perform PCR or the reverse transcriptases necessary to amplify from RNA targets. Similarly, the fluorophores used in the real-time process are quenched by substances like haem in the blood and blood itself has its own absorbance and emission spectra, the net result being that real-time PCR is considered to be unreliable in the presence of high concentrations of blood. The onus on sensitivity (particularly when using low volumes of blood) means that the blood must, by necessity, form a greater percentage of the total reaction volume in the PCR and will reach the point at which it is no longer possible to perform real-time PCR due to the opacity of the reaction.

In view of the above, PCR based diagnostics using blood samples typically require multiple steps, for instance in removing the red blood cells so that the plasma or serum could be taken into the PCR reaction, extracting the pathogen nucleic acid, and/or in extracting the resultant PCR product so that detection can be performed in the absence of the red blood cells. These multiple steps require lab equipment, a bio-safe environment and safety equipment to ensure that clinical practitioners and laboratory staff are not infected, meaning that detection is not simple, and is not suited for rapid field-based detection of pathogens.

It is a well established fact that PCR is inhibited by the presence of whole blood, there are very few peer reviewed papers on direct from blood QPCR as only very low amounts of blood can be added to a reaction before the process is completely optically inhibited. The amount of blood that can be added and the relatively small volume of the described methodologies (<50ul) means that direct from blood pathogen detection has been very infrequently described. For standard QPCR Minogue *et al* (Minogue, Timothy D et al. "Cross-institute evaluations of inhibitor-resistant PCR reagents for direct testing of aerosol and blood samples containing biological warfare agent DNA" Applied and environmental microbiology vol. 80,4 (2014): 1322-9.) described spiking spores directly into whole blood, but this was only up to 4% and critically does not describe any spinning step. There is even less in the literature concerning direct from blood reverse transcription QPCR, since there is even less literature surrounding the use of reverse transcriptases in the presence of blood. Those that do exist are centred around parasites like malaria, where large number of ribsomal RNAs can be used as a PCR target, for example there can be tens of thousands of ribsomal RNA transcripts per parasite (Taylor BJ, Lanke K, Banman SL, et al. A Direct from Blood Reverse Transcriptase Polymerase Chain Reaction Assay for Monitoring Falciparum Malaria Parasite Transmission in Elimination Settings. Am J Trop Med Hyg. 2017;97(2):533-543), or viruses like Ebola where the viral titre can be in the millions per ml of whole blood (Kavit Shah, Emma Bentley, Adam Tyler, Kevin S Richards, Ed Wright, Linda Easterbrook, Diane Lee, Claire Cleaver, Louise Usher, Jane E Burton, James Pitman, Christine B Bruce, David Edge, Martin Lee, Nelson Nazareth, David A Norwood, Sterghios Athanasios Moschos. Field-deployable, Quantitative, Rapid Identification of Active Ebola Virus Infection in Unprocessed Blood. Chem. Sci., 2017; DOI: 10.1039/C7SC03281A). The inhibition of PCR by whole blood has been shown to be multi-factorial, caused by the presence of iron containing Haem compounds, immnunoglobulins and simply due to the presence of competing cations in the blood such as calcium (Sidstedt, Maja et al. "Inhibition mechanisms of hemoglobin, immunoglobulin G, and whole blood in digital and real-time PCR" Analytical and bioanalytical chemistry vol. 410,10 (2018): 2569-2583).

Methods of lysing cells, such as blood cells, are known, for instance from WO2011157989. However, the method of WO2011157989 requires high amounts of energy to allow the required freezing and thawing cycles. Such a method is not suitable for field-based detection of pathogens using battery operated PCR machines.

US 2017/268043 relates to methods for identifying the presence or absence of a target nucleic acid, where the method comprises pelleting cellular debris and fluorescence inhibitors present within the sample and amplifying the target nucleic acid. US 2017/0239653 and US 6783993 relates to methods of nucleic acid amplification performed during centrifugation.

US 6 783 993 B1 discloses homogenizing of small-volume mixtures by centrifugation and heating.

WO2016139443 discloses methods for performing PCR directly on a blood sample. However, this method is restricted to the use of relatively low quantities of blood in each PCR reaction, meaning that the sensitivity is limited, and is also restricted to particular excitation and emission wavelengths. These wavelengths do not correlate to the majority of commonly used fluorophores, though suitable fluorophore combinations do exist such as CY5-BHQ2, HiLyte647-QXL607, limiting the potential for multiplexing.

In view of this, single-step, closed-tube PCR based methods of pathogen detection, particularly from blood samples, that are cheap, simple and suitable for field use where access to electricity is restricted, are not available. Any instruments or assays capable of processing whole blood, such as the BioFire or SmartCycler require upstream processing and by their nature are more complex and hence expensive per test, by virtue of their absolute requirement for automating the nucleic acid extraction process.

Furthermore, commonly used PCR reaction vessels are typically sealed either by a flip cap closure or in the case of plates a sealing film is the preferred sealing means. In this context caps means a lid having a diameter just less than that of the reaction vessel to be sealed, the cap is provided with a sealing flange that locks the cap in place by an interference fi, i.e. there is no means provided to prevent the cap from opening and release of the vessel contents. During PCR the cap is generally prevented from opening by pressure from the instrument itself which offers no protection to the clinical or lab worker handling large numbers of potentially deadly contaminated material every day.

Typically PCR vessels comprise a sealable, disposable well of microtitre capacity, that is a capacity less than 200µL (200 microlitres), and are formed of clear polypropylene and have a translucent cap enabling optical monitoring. Traditionally also they are simple and constructed for use in a rectangular holder which will receive 96 such vessels in a 12 x 8 array on centres no more than 10mm apart.

These typical, known reaction vessels are unsuitable for field use with dangerous pathogens, particularly because, even though the user may be wearing biologically protective clothing, he may be travelling at least a small distance carrying the reaction vessel and thus be at risk from a compromising incident. Additionally, the intended users of this technology such as the military and first responder medical personnel have communicated an absolute requirement that the biosecurity of the reaction vessel is paramount. In the normal laboratory context, testing for category 4 pathogens, such as Ebola, is first performed by the taking of blood from a venous draw. The Vacutainer containing the blood is then dipped in strong bleach to ensure that the outside is decontaminated of any virus and then high molarity (>4) guanidium chloride is added to the blood itself to completely denature any proteins present-this step renders the pathogen non-infectious.

It would be advantageous to have a method for the molecular detection of such pathogens in the blood of patients that could be used in-field, in the absence of the requirement for a laboratory or trained personnel. Further, if the molecular test could be performed directly from a much smaller volume of patient blood then the potential exposure of those performing the test could be greatly reduced.

Accordingly, there is a need in the art for the provision of methods and kits to allow safe, reliable and simple detection of pathogens.

The inventors have provided a bio-secure vessel in which reagents such as potentially deadly samples can be stored and handled safely, and which in some instances are suitable for use directly in a PCR reaction.

The inventors have also discovered a means of safely, successfully and reliably carrying out real time PCR to detect pathogens in crude samples such as whole blood. Moreover, the present disclosure includes means enabling the detection to be carried out rapidly in field conditions where aspects of the environment (resource and environment poor) provide great challenges.

### Summary of the Invention

The invention is set out in the claims.

This specification describes improved processes and methods, and a reaction vessel is also described herein, that make possible direct (reverse transcription) real-time PCR using any (visible wavelength) excitation and detection wavelength and hence renders an improvement in the number of target nucleic acid targets that can concurrently be screened for. The specification includes the methods necessary to perform the direct amplification of viral pathogens from crude samples such as crude whole blood samples in a single closed tube process without recourse to performing nucleic acid extraction, making rapid low-cost in-field diagnostics possible. The processes and methods using the reaction vessel described herein are particularly suited for use in the identification of dangerous pathogens in the field, whereby a crude sample, such as whole blood, is added directly into the reaction vessel and hence the biosafety of the user is paramount.

The applicants have discovered that by using the methods described herein it is possible to greatly increase the amount of crude sample, for example the amount of blood that can be added to a reaction. In the case of whole blood this amount is in excess of 35%. This has the combined benefits of increasing diagnostic sensitivity but also allowing the final volume of the reaction to be minimised, thus permitting more rapid thermal cycling and thereby reducing the time to detection which is vital in a point of care field setting. Additionally, it reduces the cost per test significantly by reducing the total reaction volume and hence proportionally reducing the cost of the reagents. A number of blood borne viral infections are found in remote, resource poor environments, including Lassa, CCHF, Ebola etc, and minimum time to detection, ease of use and cost per test are all vital. The WHO R&D blueprint states that simplified molecular diagnostics for the developing world must have a sensitivity of 3000 virions/ml and a cost per test equivalent to antibody-based approaches. The present invention achieves this, increasing the sensitivity to below 1000 virions/ml and reducing the cost to that of lateral flow immunodiagnostics. Assays using the described method have been shown capable of detecting as few as 15 virions per reaction, so assuming that 15ul of blood has been added this would be a final sensitivity of 1000 virions/ml. This means that a significant proportion, as much as 60%, could afford to be lost due to centrifugation and still meet the WHO target.

Where the sample is a blood sample, the applicants have discovered that some reagent mixtures, based on variables such as pH and the presence of adjuncts, can be more denaturing to the blood. The consequence of that is that the optical system described in WO2016139443 (which describes a high powered laser based spectrography based approach for multiplexed detection in the presence of whole blood) is no longer in those circumstances able to detect real-time PCR signals in blood concentrations as high as the 13% maximum stated in that application (Kavit Shah, Emma Bentley, Adam Tyler, Kevin S Richards, Ed Wright, Linda Easterbrook, Diane Lee, Claire Cleaver, Louise Usher, Jane E Burton, James Pitman, Christine B Bruce, David Edge, Martin Lee, Nelson Nazareth, David A Norwood, Sterghios Athanasios Moschos. Field-deployable, Quantitative, Rapid Identification of Active Ebola Virus Infection in Unprocessed Blood. Chem. Sci., 2017; DOI: 10.1039/C7SC03281A). The reason for this reduced performance is that in a more denaturing reagent, the blood turns from a red liquid into a "brown" colloidal suspension of denatured protein which increases the opacity of the liquid. At higher percentages of blood a dark brown colloidal suspension is formed that completely prevents the collection of optical data. Although the applicants have been able to formulate reagents capable of performing reverse transcript quantitative PCR (RT-QPCR) in the presence of as much as 40% whole blood the process is not viable as optical data can no longer be discerned. If it was possible to avoid the formation of this suspension then it would be possible to add greater concentrations of blood suspected of containing the viral pathogen and hence significantly increase the sensitivity of the process.

Plasma and serum are routinely used sample types for molecular diagnostics, both of these are formed by the centrifugation of whole blood to remove the red blood cells either after allowing the blood to form clots (serum) or using blood that has not clotted. As a centrifugation step has been performed, 2000g for 5-10 minutes being routinely used, then it is sometimes observed that a lower diagnostic sensitivity is achieved when plasma or serum is used as the sample as opposed to whole blood. This is understood to be because some of the pathogen may be contained in cell debris that is spun down. An example of this is in efforts to detect Dengue virus (Klungthong C, Gibbons RV, Thaisomboonsuk B, et al. "Dengue virus detection using whole blood for reverse transcriptase PCR and virus isolation." J Clin Microbiol. 2007;45(8):2480-5.). It means that when serum or plasma is used as the diagnostic sample type then it will no longer contain the red blood cells, white blood cells and any other component which may be spun down including cellular debris resulting from viral infection and it is this that is believed responsible for the lowered diagnostic sensitivity.

Therefore, a closed tube, direct RT-PCR method in which whole blood was added to the reaction and yet plasma was formed within the reaction vessel - by performing a centrifugation step with the absolute minimum relative centrifugal force applied for the shortest time would have the advantage of removing the red blood cells from the optical path but would leave the target pathogens and white blood cells and cell debris in suspension due to their lower mass and hence maximise diagnostic sensitivity.

### Detailed description of the invention

The present invention provides a method for the detection of a pathogen in a sample that may comprise one or more pathogens, wherein the method comprises performing RT, PCR or RT-PCR and wherein the sample comprises particulate or cellular matter;
characterised in that the method comprises:
adding the sample to a vessel that comprises PCR reaction components; and
centrifuging the sample prior to RT and PCR and not during RT or PCR within a closed vessel and wherein the vessel is the same vessel as the PCR is to subsequently be performed in,
   and
   wherein the centrifugation step is performed:
   a) at a speed and duration so as to pellet the particular/cellular material whilst leaving the pathogen in the supernatant; or
   b) wherein where the sample is a blood sample, the centrifugation is performed at a speed and for a duration so as to result in pelleting a first fraction of the sample whilst leaving a second fraction of the sample in the supernatant, wherein the first fraction comprises red blood cells and the second fraction comprises white blood cells and any viruses or bacteria present in the sample.

The invention also provides a method for diagnosing a subject as being infected with a target pathogen, wherein the method comprises the method for the detection of a pathogen according to the invention.

The specification discloses a vessel which in one disclosure comprises:
a reaction chamber portion;
a cap holder portion having a means for attaching a cap in such a way that when the cap is engaged with the vessel there are at least two points of security between the cap and the reaction chamber portion;
and a cap.

The vessel disclosed herein is suitable for use in the method of the invention.

The vessel may be any type of vessel, and may be made to contain any type of sample. For example the vessel may be suitable for containing a liquid, solid or gas. In a preferred embodiment the vessel is suitable for containing a liquid. The vessel may be suitable for containing both a liquid and a solid, for example may be suitable for containing a lyophilised powder of, for example, PCR components, to which a liquid is subsequently added.

The vessel may be a re-useable non-disposable vessel. Since the vessel may be used for the detection of harmful pathogens, in a preferred disclosure the vessel is a disposable vessel. For the vessel may be is made from a material that is relatively cheap. The vessel may made from a material that is easy to destroy, for example the material can be incinerated along with potentially harmful pathogens.

By disposable we include the meaning of single-use. For example, in once the cap is attached to the reaction chamber, it may become impossible to take off without destroying the vessel. In other disclosures the cap can be removed, for example by lifting a tab that may be located on the cap, without destroying the vessel. By single-use we include the meaning of a vessel that is intended to be disposed of after a single use, for example because it may contain one or more pathogens.

The vessel may be made from any type of suitable material, for example may be made from a polymer such as a carbon loaded polymer. Injection mouldable thermoplastic polymers capable of tolerating the maximum 110°C temperatures that a thermal cycler is capable of, for example polypropylene, are considered to be suitable materials.

Where the vessel comprises a carbon loaded polymer, in some embodiments the carbon loading is at least 30%, 35%, 40%, 45%, 50%, 60%, 65%, 70% by weight. Preferably the carbon loading may be 60% by weight.

The reaction chamber may preferably be formed of polyurethane, and may be loaded with carbon in the range of at least 30%, 35%, 40%, 45%, 50%, 60%, 65%, 70% carbon by weight. The reaction chamber may preferably be formed of polyurethane loaded with 60% carbon per weight.

The vessel may comprise different materials, for example the cap portion may be made from a different material to the reaction vessel portion.

Preferably, the vessel is 2 shot moulded. The cap holder portion may be made of polypropylene due to the requirement for light transparency and its ability to act as a thermal insulator. The base may be thermally conductive but the top is not.

The cap holder portion and the reaction chamber portion may be a single entity, for example wherein the cap holder portion is formed to the reaction chamber portion.

The vessel may be suitable for field use. By field use we include the meaning of use in a non-standard laboratory setting. In some instances, by field use we mean for use in an environment where electricity is limited, or available only by battery. By field use we also include the meaning of situations wherein the clinical practitioner, laboratory scientist, or other person who handles the vessel is not equipped with suitable safety equipment for the nature of the substance for which the vessel is to be used. For example, in some instances the vessel is to be used to detect highly pathogenic viruses and bacteria and will typically be used to house a sample from a subject, or an environmental sample and in certain situations, such as in field use, the person handling the vessel may be equipped with only very basic safety equipment.

The vessel may be considered to be a bio-secure vessel. The skilled person will appreciate what is meant by the term bio-secure. In some instances bio-secure means that none of the pathogen contained within the vessel is able to escape from the vessel, and so the person handling the vessel cannot be exposed to any pathogen contained within. In some instances a bio-secure vessel has a lid or a cap which locks. Preferably a bio-secure vessel is made of a material that is crush proof, such as a carbon loaded polymer. In some instances the bio-secure vessel also has 2 points of security in sealing. The vessel described herein may be suitable for use in an amplification reaction, for example a PCR reaction, optionally suitable for use in reverse transcription (RT) PCR, optionally for use in quantitative (q) PCR or RT-qPCR. Vessels suitable for use in such reactions typically have thin walls of around 0.5 to 0.8mm in thickness and made of carbon loaded polymer. This does 2 things 1) makes it gain and lose heat faster 2) reduces lag between reaction vessel holder/tube/liquid contents.

The skilled person will understand what is meant by qPCR. In this case the reaction contains primers, or groups of primers when the target pathogen has high levels of sequence heterogeneity, such as Lassa, and a probe which is sequence specific to the target of interest. This could be a hydrolysis probe whereby the 5' end is labelled with a fluorophore and the 3' with a quenching moiety - during amplification the probe is hydrolysed by the enzyme and as such fluorescence increases cycle on cycle. Excitation means is provided and the resulting emission is captured through a window in the vessel, which in some embodiments is located in the cap.

The vessel may be suitable for the identification of pathogens, and in particular those pathogens that are considered to be capable of causing severe disease. For example, in the vessel may be suitable for the containment and identification of pathogens that fall into categories 3 and 4 of the Classification of Biological agents, National Institute for Public Health and the Environment, RIVM Letter Report 205084002:

| **Classification** | **Ability to cause disease in humans (virulence)** | **Hazard to workers** | **Spread to the community (transmission)** | **Effective prophylaxsis or (treatment)** |
|---|---|---|---|---|
| Category 1 | Unlikely | No hazard | Not applicable | Not applicable |
| Category 2 | Likely disease | Potential hazard | Unlikely | Available |
| Category 3 | Likely severe disease | Serious hazard | Likely | Available |
| Category 4 | Severe disease | Serious hazard | High risk | Not available |
| Similar definitions are also used by other countries and international organizations: | | | | |
| WHO | Laboratory Biosafety Manual, 3rd Edition (2004) | | | |
| PHAC | Laboratory Biosafety Guidelines, 3rd Edition (2004) | | | |
| CDC | Biosafety in Microbiological and BioMedical Laboratories (BMBL), 5th Edition (2009) | | | |
| ***Note. An extensive overview of the criteria of biological agents is presented on the website of the American Biological Safety Associaion - ABSA*** | | | | |

The pathogens may be selected from but not limited to:
a) the group consisting of viruses, bacteria, fungi, parasites; and/or
b) Class 4 or 3 pathogens; and/or pathogens that cause the diseases selected from the group consisting of:
c) Malaria, HIV, viral hepatitis, soil transmitted helminth parasitic infections;
d) Viral haemorrhagic fevers selected from the group consisting of Ebola, Lassa fever, Marburg virus disease, Rift valley fever, Congo fever; and/or
e) Japanese encephalitis, Dengue, Zika, Chikungunya, yellow fever.
f) Veterinary diseases with a viraemic component, including but not limited to PPRV, FMDV, BTV, Newcastle disease, Swine Flu, BVDV.

Since the vessel may be used with qPCR or RT-qPCR, the vessel may comprise a window through which is it possible to both excite the fluorophore(s) associated with the qPCR/RT-qPCR and to capture the resultant emitted light.

Preferably the window is a translucent window, and is suitable for the excitation of and/or detection of emissions from, fluorophores located within the vessel.

The window may be located anywhere in the vessel, for example, in the cap, the side or the base. However, preferably the window is located in the cap portion of the vessel since this allows a greater variety of sample types to be used in the RT-PCR. For example, where blood is used as the sample in the PCR reaction, the blood may be intentionally spun down to the base in accordance with the methods of the invention, precluding detection of the resultant PCR products via the base.

It is important that the vessel comprises at least two points of security, to prevent the contained sample, for example the pathogen, from escaping. The skilled person will understand that a standard vessel used in PCR reactions only has a single seal. In this case, if the lid is crushed or the vessel is dropped then there is a high chance of the contents escaping from the vessel. The at least two points of security of the vessel may be arranged such that in order for the contents of the vessel to bypass the at least two points of security and escape from the vessel, the contents have to a) pass through the at least two points of security; b) change direction multiple times; and c) fit through very small spaces. This arrangement makes it impossible for the contents of the vessel, such as liquids to get out of the vessel. Preferably the vessel also comprises a lock feature that keeps the at least two points of security, for example at least two seals compressed and functional.

The point of security may be the result of an interference fit between, for example, the cap and the vessel. One or both of the points of security may be a gasket, for example is an O-ring seal.

At least one of the points of security may comprise a seal, for example an O-ring, between the cap and the vessel.

In some instances, both, or at least two of the points of security comprises a seal, for example comprises an O-ring between the cap and the vessel.

In some instances at least one of the seals is located at the top of the cap holder portion.

At least one of the seals may be located at the at the base of the cap.

The vessel may comprise at least two seals between the cap and the vessel and wherein one of the seals is located at the top of the cap holder portion and wherein one of the seals is located at the base of the cap.

In addition to the seals such as O-ring seals the vessel may comprise interference fit at multiple points.

As discussed above, the vessel may comprise a locking means designed to prevent easy or accidental separating of the cap from the reaction chamber portion.

Suitable locking means will be known to the skilled person. The locking of the cap to the holder may comprise a "clip-over" device which may be achieved by resilient means between a wing on the cap and a "ramp and step", for example on a wing, preferably on the holder portion as distinct from the cap.

It is considered important that the cap fits securely to the vessel. One means of achieving this is through the use of a screw thread assembly. Accordingly, the cap may comprise a screw thread with the cap holder portion comprising the appropriate complementary screw thread, enabling the attachment of the cap to the cap holder portion. In some instances the screw thread assembly is key and helps to pressurise the seals and so should be made of a non-brittle material, such as polypropylene.

It will be appreciated that where the cap/vessel assembly comprises a complementary screw thread, it should be arranged so that it is easy to use and requires minimal effort to securely attach the cap to the vessel. Accordingly, in some instances the cap requires less than 5, optionally less than 4, 3, 2, or 1 turn on the screw thread to securely attach the cap to the cap holder portion.

Overtightening of the cap to the vessel can result in weaknesses being introduced into the structure of the vessel. Accordingly, in some instances the vessel comprises a stop arranged to prevent overtightening of the cap.

The vessel may also comprise further useful features, such as a wing on the cap holder portion for attaching a label to or for inscribing thereon, optionally for attaching an identifier label or inscribing an identifier.

The vessel may be suitable for being held in a centrifuge, and suitable for use in the methods of the invention described below. Accordingly, in use the vessel may be able to withstand gravitational force as applied via centrifugation. The skilled person will understand this need for robustness in the reaction vessel assembly and is able to select suitable materials accordingly.

Where the window is incorporated into the cap of the vessel, it is considered advantageous if the vessel is constructed to allow heating of the cap so that condensation does not form obstructing excitation/emission of the fluorophores when used with qPCR, for example. One way of heating the caps during amplification is to contact the caps with a heated lid. Accordingly, in some instances the cap of the vessel is flat so as to enable contact with a heater.

The reaction vessel may be of any size or capacity. However, the capacity of the vessel is typically suitable for use in a PCR/amplification machine and typically has a capacity of 1000ul or less, for example less than 900ul, 800ul, 700ul, 600ul, 500ul, 400ul, 300ul, 250ul, 200ul, 150ul, 100ul, 50ul or less than 20ul. Preferably the vessel has a capacity of around 200ul or 200ul. Accordingly in some instances the vessel has a micotitre capacity.

The reaction vessel may be between 3-5 cm in length and where the vessel comprises a wing or wings, is between around 3 - 4 cm in breadth.

The vessel may comprise or contain PCR reaction components, for example comprises or contains one or more of a polymerase or PCR primers, for example wherein the reaction components are lyophilised.

As set out in the claims, provided herein is a method for the detection of a pathogen in a sample that may comprise one or more pathogens, wherein the method comprises performing RT, PCR or RT-PCR and wherein the sample comprises particulate or cellular matter;
characterised in that the method comprises:
adding the sample to a vessel that comprises PCR reaction components; and
centrifuging the sample prior to RT and PCR and not during RT or PCR within a closed vessel and wherein the vessel is the same vessel as the PCR is to subsequently be performed in,
   and
   wherein the centrifugation step is performed:
   a) at a speed and duration so as to pellet the particular/cellular material whilst leaving the pathogen in the supernatant; or
   b) wherein where the sample is a blood sample, the centrifugation is performed at a speed and for a duration so as to result in pelleting a first fraction of the sample whilst leaving a second fraction of the sample in the supernatant, wherein the first fraction comprises red blood cells and the second fraction comprises white blood cells and any viruses or bacteria present in the sample.

Also disclosed herein is a method for preparing a sample for PCR, optionally for qPCR, optionally for RT qPCR, wherein the method comprises centrifuging the sample a vessel and wherein the vessel is the same vessel as the PCR is to subsequently be performed in.

There are significant advantages if the method of the invention is performed in the vessel disclosed herein, as will be apparent to the skilled person, such as reduction in the risk of exposure to the potentially deadly contents. However, it is possible to perform the method of the invention in a vessel that is not the vessel disclosed herein.

In one embodiment of the method of the invention, it is preferred if no part of the sample is removed from the vessel prior to PCR being performed, for example where once the sample is added to the vessel, no material is removed from the vessel prior to or during PCR. In the same or different embodiment it is also preferred if no part of the sample is removed from the vessel following the completion of PCR, for example in some embodiments once the sample is added to the vessel, no material is removed from the vessel:
a) prior to PCT; and/or
b) during PCR; and/or
c) following PCR.

For example, in preferred embodiments the detection of the PCR product is performed within the vessel without the vessel needing to be opened and material transferred elsewhere. In these embodiments the risk of exposure to the contents of the vessel is reduced.

In a preferred embodiment, the sample is a crude sample. By a crude sample we include the meaning of a sample to which no additional steps have been applied following the obtaining of the sample. For example the crude sample may be a crude biological sample, such as a whole blood sample, faeces, urine, CSF, eluate from swabs taken from a subject, optionally taken from the eyes, ears, nose or mouth. A crude biological sample is considered to be any sample taken directly from an organism. The crude sample may also be a crude environmental sample. By a crude environmental sample we include the meaning of samples such as food samples, swabs from surfaces and any other sample type that isn't taken directly from an organism (in which case it would be considered to be a crude biological sample).

The crude sample may also be a sample to which minimal processing steps have been applied following obtaining the sample, such as in the preparation of plasma and serum from whole blood, or an eluate from a wash of a swab, for example for pathogens that are not highly infectious to the operator, for example when used for the detection of veterinary pathogens in the field.

The crude sample will typically comprise particulate or cellular material which in some embodiments may be considered to interfere with the excitation of fluorophores and/or capture of the emitted wavelengths and may interfere with the choice of suitable fluorophores. For example where the sample is blood the choice of fluorophores is, prior to the present invention, limited. By performing the centrifugation step, the crude sample, for example the blood, is removed from the optical path making it possible to detect a wider range of fluorophores if a suitable optical system is utilised. Without centrifugation it is not possible, for example, to use green dyes such as fluorescein because optical quenching is up to 90% of the signal. In the clarified top layer after centrifugation this is reduced to the region of 30% and hence real-time PCR by all wavelength dyes is possible using the present methods and vessels.

The sample used in the method of the invention comprises particulate or cellular matter.

It is considered preferable from a safety point of view if the sample is obtained and added directly to the vessel (which may or may not be a vessel as disclosed herein). The skilled person will of course appreciate that following obtaining the sample the sample may be stored for some period of time, for example at cold temperatures, prior to adding to the reaction vessel.

As discussed above, the sample is a sample (crude or otherwise) that may comprise one or more pathogens, optionally may comprise one or more bacterial species, virus, fungi, parasites for example. Preferences for the pathogen are described above in the context of the vessel and apply here.

The crude sample, for example the crude whole blood sample in a patient infected with a pathogen such as a viral pathogen will not have an identical concentration of the virus in any given volume of blood. This is because for some diseases the pathogen for example the virus may be associated with cell debris rather than being free in the sample, for example in free blood. Thus, there is a benefit in processing the highest possible volume of patient blood in the direct RT-PCR method - both by maximising the amount of target pathogen present but also by virtue of being able to minimise the effect of the random distribution of the pathogen within the patient's blood, a larger sample having a better chance of containing a representative titre.

In one embodiment, it is not considered to be advantageous to operate the direct method in volumes larger than 200ul, since the background fluorescence rises considerably and gives less signal-noise ratio in the data, additionally the actual concentration of pathogen RNA/ul is reduced by virtue of the greater volume.

The applicants have discovered that the process of bringing the sample for example the red blood cells to the base of the reaction vessel while leaving the virions and white blood cells in the upper solution has an additional benefit to the process - the reduction of enzymatic process inhibition caused by the presence of whole blood. The pellet of sample for example red blood cells "cooks" under the thermal denaturation steps in PCR and in doing so as a compacted lump, reduces the amounts of inhibitory compounds released into the reaction when compared to being present in the form of individual free floating cells in the whole blood suspension.

By centrifuging the sample prior to or during RT or PCR, the sample is considered to be pulled out of the way of both the window (for excitation/emission), and is also considered to reduce the amount of sample that can leach components into the PCR mix that may be inhibitory to the reaction. For example, upon heating, blood cells are considered to release inhibitory substances. However, following centrifugation, it is only the top layer of blood cells, i.e. those in contact with the PCR mix that are considered to release the inhibitory substances into the mix. Accordingly, the centrifugation step allows a larger amount of sample per reaction volume, for instance a larger amount of blood, to be used since a) the blood no longer inhibits excitation/emission; and b) a lower amount of inhibitory substances are released from the blood.

Accordingly, in one embodiment, the sample comprises a substance, or releases a substance, for example releases a substance upon heating, that is inhibitory to PCR.

The sample, crude or otherwise but particularly in crude samples, is expected to comprise both particular /cellular matter which is largely considered to be non-useful in the amplification reaction, but also comprises the target pathogen, for example bacteria or viruses. Accordingly in one embodiment the centrifugation step is performed at a speed and duration so as to pellet the particular/cellular material whilst leaving the pathogen in the supernatant. The skilled person will understand how to choose these parameters based on the size, weight and density of the relevant components.

Accordingly, in some embodiments the centrifugation is performed at a speed and for a duration so as to result in pelleting a first fraction of the sample whilst leaving a second fraction of the sample in the supernatant.

White blood cells have a density of 1.080 g/ml but red blood cells are more dense at 1.110g/ml and this, combined with their large size ensures that they are pulled down further under centrifugation than the white blood cells. Accordingly, when samples containing blood are centrifuged it is well established that the white blood cells, being less dense, come to rest upon the layer of red blood cells - this being called the buffy coat layer. A number of viral pathogens, for example Chikungunya in humans and PPRV in animals, actually use the host white blood cells as their site of replication. This is evidenced by the fact that for detecting some viruses this buffy coat layer is actually the optimal sample type, since it contains the infected white blood cells while removing the diluting plasma and the (process) inhibitory red blood cells. (Madani, T.A., Abuelzein, ET.M.E., Azhar, E.I. et al. Arch Virol (2012) 157: 819. https://doi.org/10.1007/s00705-012-1237-7). The applicants have been able to demonstrate that it is possible to manipulate these two facts; that the white blood cells have lower density than the red blood cells and that they can contain the viral target (for certain pathogens) - to perform the centrifugation step such that the optically and enzymatically inhibitory red blood cells may be spun to the base of the vessel and yet the significant proportion of white blood cells may be left in suspension in the plasma and hence maximise sensitivity for those viruses where replication occurs within the white blood cells such as Chikungunya (Wikan, Nitwara et al. "Comprehensive proteomic analysis of white blood cells from chikungunya fever patients of different severities" Journal of translational medicine vol. 12 96. 11 Apr. 2014, doi:10.1186/1479-5876-12-96).

Furthermore, Virions are more dense than either red or white blood cells, ranging between 1.2 and 1.4g/ml, but critically they are less than a thousandth of the size of the red and white blood cells. Migration under centrifugal force is governed by two main factors, density and size of particle - smaller particles take longer for the gravity to move them down a density gradient. This means that centrifugation at a relatively low speed can pull down the red blood cells while leaving the white blood cells (by virtue of lower density) and the virions (by virtue of their small size) in suspension. Thus, it is possible to remove the optical inhibition resulting from the presence of the red blood cells, reduce the inhibition of the enzymatic process, while concurrently maximising diagnostic sensitivity that would otherwise be lost if serum or plasma was used as a sample input - since the white blood cells remain in the closed tube reaction vessel.

Accordingly this method of, in essence, making plasma in a closed reaction vessel and then performing direct RT-QPCR on it - makes possible a closed-tube, extraction free method for testing patients for the presence of viral infection at the point of need in an emerging disease outbreak situation.

A field strength (RCF) of 500g for a brief 30 second spin (other RCFs and durations are of course also acceptable, as discussed herein) has been experimentally tested and found sufficient to spin the red blood cells down while leaving the majority of white blood cells still in suspension. In essence this provides a closed tube method for the direct detection of viral pathogens from blood that removes the optically and enzymatically inhibitory red blood cells but leaves the white blood cells and patient plasma in the body of the amplification reaction. The separation is both a function of centrifugal field and time and as such it could be envisaged that a number of combinations could deliver the required removal of red blood cells while leaving the white cells and virions in suspension, as discussed herein.

In the case where the sample is whole blood, the first fraction comprises the red blood cells, while the second fraction comprises the white blood cells along with any target pathogens, such as any viruses or bacteria present in the sample. This is because some pathogens are known to target white blood cells. White blood cells are less inhibitory to the amplification reactions and their presence in the supernatant, along with any potential pathogens that they may harbour, is considered to improve the sensitivity of the reaction by making them accessible to the amplification reagent.

In some embodiments the centrifugation is performed at a speed and for a duration so as to result in the formation of plasma.

Centrifugation prior to PCR is often used to ensure that the amplification reagents are at the base of the reaction vessel, however this is not performed for any technical reason other than that described above. There are papers covering direct PCR from blood that call for a centrifugation step after PCR is complete but that is so that the clear supernatant can be transferred to a secondary process such as electrophoresis - as an example the manual for the Agilent Suredirect PCR kit recommends a 5 minute high rcf centrifugation after PCR so that a clear supernatant can be run on a gel. In terms of real-time PCR direct from blood the background art is more scant. The applicants have described an optical approach to performing real-time PCR in high concentrations of blood (WO2016139443). In this, data is provided demonstrating that the presence of whole blood introduces inhibition of fluorescence by as much as 90% in certain wavelengths and that this in part explains the lack of publications in the field of direct real-time PCR in samples containing whole blood. Direct from blood RT-PCR to detect pathogens is not performed because of the optical inhibition, process inhibition (from haem, immunoglobulins and the presence of excess calcium and other minerals in the blood) and because the pathogen nucleic acid must first be rendered amplifiable. The applicants have solved both the optical issue and developed a method to render the pathogen nucleic acid contained in the whole blood sample amplifiable (WO2011157989.; Kavit Shah, Emma Bentley, Adam Tyler, Kevin S Richards, Ed Wright, Linda Easterbrook, Diane Lee, Claire Cleaver, Louise Usher, Jane E Burton, James Pitman, Christine B Bruce, David Edge, Martin Lee, Nelson Nazareth, David A Norwood, Sterghios Athanasios Moschos. Field-deployable, Quantitative, Rapid Identification of Active Ebola Virus Infection in Unprocessed Blood. Chem. Sci., 2017; DOl: 10.1039/C7SC03281A) is also relevant.

In some embodiments, for example where the sample is a blood sample, the centrifugation is performed at less than 1000g, optionally between 100g and 1000g, between 200g and 900g, between 300g and 800g, between 400g and 700g, optionally between 500g and 600g. In the same or different embodiments the centrifugation may be performed for less than 60 seconds, optionally between 5 and 60 seconds, optionally between 10 and 55, 15 and 50, 20 and 45, 25 and 40, 30 and 35 seconds. In a preferred embodiment, for example where the sample is a whole blood sample, the centrifugation is performed at 500g for 30 seconds. Centrifugation at too high a relative centrifugal force or for too long can reduce process sensitivity, either by lysis of some proportion of the red blood cells or by being sufficient to pull the white blood cells or the target pathogen, out of suspension. 500g for 30 seconds is considered to force the red blood cells to the base of the reaction vessel, without damage, while the target pathogens, for example target virions - having much lower mass - remain in suspension and hence are susceptible to direct RT-PCR detection.

It can be envisaged by those in the field that differing combinations of relative centrifugal force and or time could be applied to give the same result. However the applicants have experimentally determined that 500g for 30 seconds represents the shortest time (vital for rapid diagnostics) that the separation of red blood cells can be achieved while leaving the target pathogens in suspension and hence liable to RT-PCR based detection by this method.

The centrifugation could be performed straight after the sample, for example the crude sample, for example the blood, has been added to the vessel but after a mixing process, for example an automated mixing process.

Following the centrifugation step an optional freeze-thaw cycle may be performed (EP2585581), to render the target pathogens directly amplifiable. Directly performing RT-QPCR on the target pathogen of interest from the crude sample in a closed tube process that does not require any separate nucleic acid extraction step.

Prior to adding the sample, the vessel comprises PCR reaction components, optionally comprises any one or more of polymerase and/or PCR primers. This is considered advantageous since it means that as soon as the sample is added the vessel can be sealed, reducing risk of exposure.

For in-field use it is preferable that the centrifugation, for example 500g for 30 seconds, takes place before the RT and/or PCR process begins - because this ensures the least processing steps for a non-expert operator. Spinning after the lysis, RT or first few cycles has the disadvantage that the reaction vessel must be removed from the apparatus and placed into the centrifuge. Moreover, in circumstances where the brown colloidal suspension forms, this suspension has a much higher Relative Centrifugal Force required to spin it to the base of the vessel. Additionally there will be a higher concentration of PCR inhibitors present as the red blood cells will have lysed. The applicants have discovered that the optimal RCF is 2000g for 30 seconds to spin down the brown colloidal suspension, some 1500g more than spinning prior to beginning the process, but that any centrifugal force would be suitable if the correct time was utilised.

In the method of the invention, the centrifugation takes place prior to commencement of RT and PCR.

In some embodiments, centrifugation may optionally take place after the RT step (if present) but prior to PCR.

For example, in a particular embodiment where the pathogen comprises an RNA target nucleic acid, for example where the pathogen is an RNA virus, for a quantitative method it may be preferable that the centrifugation takes place after the virus has been lysed and the more stable cDNA created from the viral RNA genome.

As described above, in a particularly advantageous embodiment, the method takes place in a vessel as disclosed herein.

In some embodiments the PCR is qPCR or the RT-PCR is RT-qPCR. In some embodiments of qPCR or RT-qPCR the excitation wavelength used to excite the fluorophore associated with the qPCR is between 630nm-645nm, optionally between 633nm-642nm; and/or the emitted light is collected at a wavelength of between 650nm-750nm. The skilled person will understand which fluorophores are appropriate for use with these parameters. In one embodiment the PCR uses a 2 colour system with LED excitation at 475nm and 635nm and collection of the emission at 400-900nm using a dual band pass filter with windows of 520-580nm and 660-750nm.

In some embodiments, the method is a closed-tube method of performing PCR wherein the sample is prepared according to disclosure. Preferences for features of this method are described above, for example the method may be performed in a vessel according to the disclosure, for example on a crude sample as defined above.

In one embodiment the closed-tube method removes multiple liquid transfer steps, reducing the time taken to perform the analysis while reducing exposure of the operator to the pathogen. In one embodiment the method is essentially a method for making plasma in a closed tube and then directly amplifying from the plasma. As discussed above, in this way no nucleic acid is removed from the sample and all pathogens, for example virions or bacteria, are in the reaction vessel and lyse, making their associated nucleic acid available for amplification and subsequent detection.

In any of the methods described herein, the sample for example the crude sample for example whole blood may make up at least 5% of the PCR reaction volume, optionally at least 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34% or 35% by weight of the reaction volume, or greater. In one embodiment the sample makes up 13% by weight of the reaction volume.

As set out in the claims, the disclosure provides a method of pathogen detection, for example by using any of the methods or vessels of the disclosure. In one embodiment the method comprises detection of the presence of the amplification product. In a further embodiment, detection of the amplification product indicates presence of the pathogen.

Also provided herein is a method for diagnosing a subject as being infected with a target pathogen, wherein the method comprises any of the methods provided herein.

The skilled person will understand that the methods of the invention lend themselves to being provide as a kit, or a kit of parts. For example a kit may comprise a reaction vessel according to the invention and any one or more of
PCR primers;
Polymerase;
a resuspension buffer;
positive and/or negative control samples;
pipettes.

Consistent with the method of the invention, in some embodiments the method comprises the detection of viral pathogens from nasal, ocular, nasopharyngeal, oral and similar swab samples from humans and animals in field. The vessel (for example a vessel as disclosed herein) is provided to the user containing an extraction buffer, this could optionally be added if none is provided. A swab sample is provided from the patient in order to collect exuded virions onto the swab. The swab is added directly into the reaction vessel by breaking/cutting the end off. Preferably this will be a nylon flocked swab although cotton has been used. The swab is subjected to a heating step in the range of 70-82C to lyse the virions and release the virus-this action is encouraged by the buffer. Optionally the vessel contents can be cyclically frozen/thawed in order to increase the fraction of virions which are lysed. In one embodiment the extraction buffer actually comprises the amplification reagents, for example an rt-qpcr mix. For other targets, such as nasal swabs, the sample can be inhibitory to the process and as such a 2-step approach is required where the buffer is a pH stabilised solution containing detergent-the virions are lysed into this an then a small proportion is transferred to a 2^{nd} vessel that contains the amplification reagents for performing the final test.

In some embodiments of the method of the invention the method comprises detecting viral pathogens direct from blood.

The blood in certain reagent sets can denature and form a thick brown gelatinous precipitate. Clearly, this interferes with the optical interrogation of the sample. The applicants have previously described an optical detection approach based on high powered far red excitation, past 600nm, and a collection system based on spectroscopy such that multiplexed detection can be performed in the presence of blood. Relatively low relative centrifugal force, for example 10g, can be applied to the reaction such that over the course of the 45 cycles of PCR the brown precipitate described above has migrated to the base of the vessel. This enables an optical system placed horizontally to interrogate the vessel by virtue of being centred in the resulting clear liquid at the top o the vessel once the blood has spun out of the way. In order to run this process the vessel must continually be spun during the course of the PCR. Practically this is achieved by having the vessel holder on an arm attached to a stepper motor. The weight of the vessel is counter-balanced and the vessel spins continually generating a gravitational field in the range of 10-50g and this is capable of pulling the precipitate to the base of the vessel reliably by cycle 21 of the PCR- the expected range of a positive is 25-35 cycles and so there is sufficient time for a stable baseline of fluorescence before the PCR begins.

In the method of the present invention the sample is not centrifuged during RT or PCR.

In some embodiments of the present invention, a small centrifuge is used to pull the blood to the base of the vessel before the process starts and therefore the precipitate will automatically form at the base of the vessel. An advantage of this approach is that the proportion of the red blood cells that lyse during the reaction is greatly reduced and so the levels of inhibitors released is concurrently lower. As a result much greater percentages of blood can be added without inhibiting the process- without spinning the maximum tolerated by the reaction is 15% blood and with spinning this has been as high as 35%. It should be noted that for some viral pathogens, for example Chikungunya, the sensitivity may actually be reduced by this approach even though greater volumes of blood have been added. The reason being that this virus actually replicates in white blood cells and these will be spun down with the red blood cells and hence make it less likely that all virions are susceptible to lysis and amplification. It is common in this field to use either plasma or serum as the sample input in order to avoid having to process the more difficult blood sample in the laboratory. Serum is formed by allowing the blood sample to coagulate at room temperature and then spinning the clot to the base of the vessel, plasma is separated by directly centrifuging the sample and commonly this takes place for 5 minutes at 2000g. the blood cells will be spun to the bottom of the vessel and the serum or plasma supernatant is then used for nucleic acid extraction, in the case of viral pathogens this will be an RNA extraction. The applicants have discovered that the closed tube method described above is in effect a closed tube, combined method of making plasma and subsequently amplifying the viral pathogens directly-this completely removes the requirement for nucleic acid extraction and removes the need for multiple liquid handling steps or the titre losses known to be inherent in the making of plasma or serum.

In one embodiment, the method provided herein comprises the following steps.
1. Take a reaction vessel into which the components of a sequence specific amplification reaction have previously been lyophilised-this will include all components such as enzyme, sequence specific primers and probes and a suitable buffer
2. Resuspend the dried reaction with a specific volume of water
3. Add the blood sample direct into the vessel
4. Mix the reaction, preferably with a vortex.
5. Seal the lid on the reaction vessel
6. Spin the reaction vessel in the order of 500g for 30 seconds
7. Place the reaction vessel into the instrument
8. Virus is directly lysed and amplified in the reaction vessel, this can include the freeze-thaw process or be driven by the reagents themselves
9. Identify the presence of a viral pathogen sequence by means of a positive real-time PCR

The listing or discussion of an apparently prior-published document in this specification should not necessarily be taken as an acknowledgement that the document is part of the state of the art or is common general knowledge.

Preferences and options for a given aspect, feature or parameter of the disclosure should, unless the context indicates otherwise, be regarded as having been disclosed in combination with any and all preferences and options for all other aspects, features and parameters of the disclosure. For example, disclosed herein is a method of preparing a blood sample for RT-qPCR wherein the method comprises centrifuging the sample following the RT step. Also disclosed herein is a method of preparing a faecal sample for PCR wherein the method comprises centrifuging the sample prior to RT-PCR. The disclosure also provides a method of diagnosing a subject as being infected with a class 4 pathogen, wherein the method comprises centrifuging a blood sample taken from a subject and added directly to a vessel of the disclosure.

### Figure Legends

Figure 1 is a side elevation diagram of a reaction vessel assembly;
Figure 2 is an isometric side elevation of a reaction vessel assembly;
Figure 3 is a plan view of a reaction vessel assembly;
Figure 4 is an isometric cross-sectional diagram of a reaction vessel assembly.
Figure 5 - further images of a vessel according to the disclosure.
Figure 6 - Standard QPCR reagents before and after PCR without spinning
   A) shows blood added at 15% reaction before QPCR and B) after QPCR, where the reaction has gone increasingly opaque. Data from samples that have not been spun are shown in figure 8- and show that real-time PCR can no longer reliably performed at this blood percentage.
Figure 7 - Standard QPCR reagents spun before and after QPCR
   Shows blood added at 15% before (A) and after QPCR (B) when using standard reagents. In this case the samples have been spun prior to qPCR.
   Data from samples that have been spun are shown in figure 8- and show that real-time PCR can now be reliably performed at this blood percentage.
Figure 8
   Lanes A and B are RT-PCR of 106bp Ebola amplicon at 15% whole blood with (A) and without (B) spinning.
   Lanes C and D are the same RT-PCR at 22% whole blood treated in the same way. Lanes E and F are duplicates at 32% whole blood that have been spun prior to RT-PCR Lane G is the identical reaction that has not been spun prior to RT-PCR
   Lane H is 50bp ladder
   Spinning has reduced inhibition at 32% blood where spun performs the reaction but unspun fails.
   Also note no less of sensitivity resulting from the spinning-since the virus and white blood cells remain in suspension.

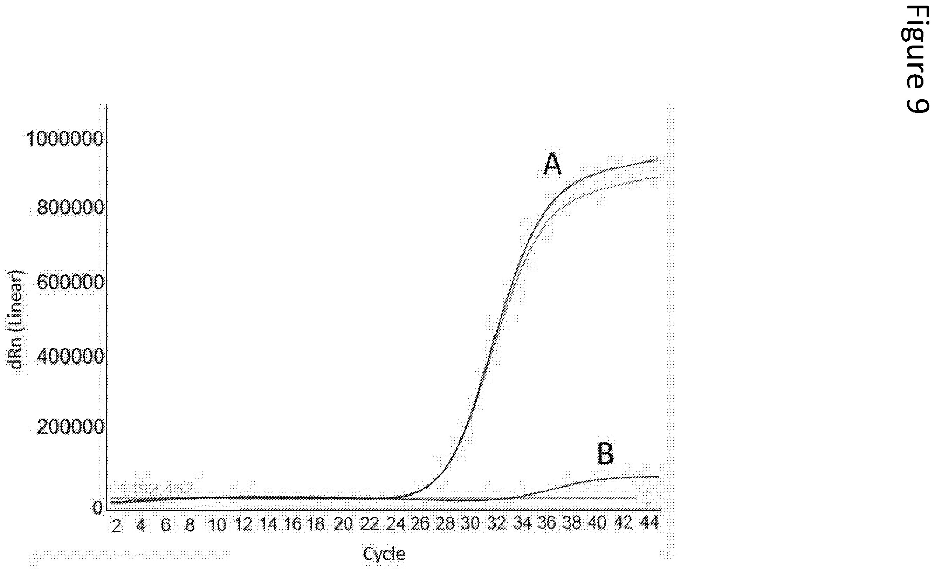

Figure 9 - RT-qPCR at 22% whole blood detecting a 106bp amplicon from the Ebola genome. A) shows 2 duplicates that have been spun at 500g for 30 seconds prior to the reaction and B) shows 2 duplicates that have not been spun. Offset of multiple cycles, some due to inhibition from lysed red blood cells but the majority due to a decrease in optical signal of 90%.

Earlier gels show that the RT-PCR result is similar but that qPCR cannot be performed due to the optical inhibition and increased inhibition from their greater proportion of lysed red blood cells.

### Examples

### Example 1

Below is provided one exemplary method of performing the methods of the disclosure:
1. The user is provided with a reaction vessel containing lyophilised reagents for the target of interest, there being an optical window in either the reaction vessel or a lid therefor.
2. The user resuspends the lyophilised reagents with a supplied resuspension buffer
3. The user adds a specified volume of whole blood (for example 20µl in a 100µl total reaction volume)
4. The lid is replaced on the reaction vessel and irreversibly sealed
5. The sample could be optionally centrifuged at this juncture - to minimise the amount of inhibitor required in the reaction even though some target pathogen may be removed into the pellet. Centrifuging at this stage requires a centrifugal force of the order of 500g for 30 seconds to achieve the desired separation.
6. The sample undergoes a lysis process - this can include
   a) Cyclical freezing and thawing
   b) And/or heating the sample to in excess of 70°C, preferably this being 95°C for 1 second
   c) This step can include an optional mixing step (PCTGB2018000011)
7. At this juncture the viral pathogen will be lysed and there will be free RNA in solution
8. The sample can be optionally spun at this juncture (if it has not already been so) PRIOR to the reverse transcription taking place, a disadvantage is that RNA is more labile but it would make the RT possible in a lower inhibitor concentration (because the red blood cells will have been spun down)
9. Preferably 2 cycles of PCR will be performed before the centrifugation, the first cycle creating double stranded DNA and the 2^{nd} doubling the amount of DNA target
10. The range of centrifugation required is 30s to 1 minute at 1-2000g- but pausing a RT-QPCR and spinning for any time/gravity field strength is envisaged by this method.

### Example 2

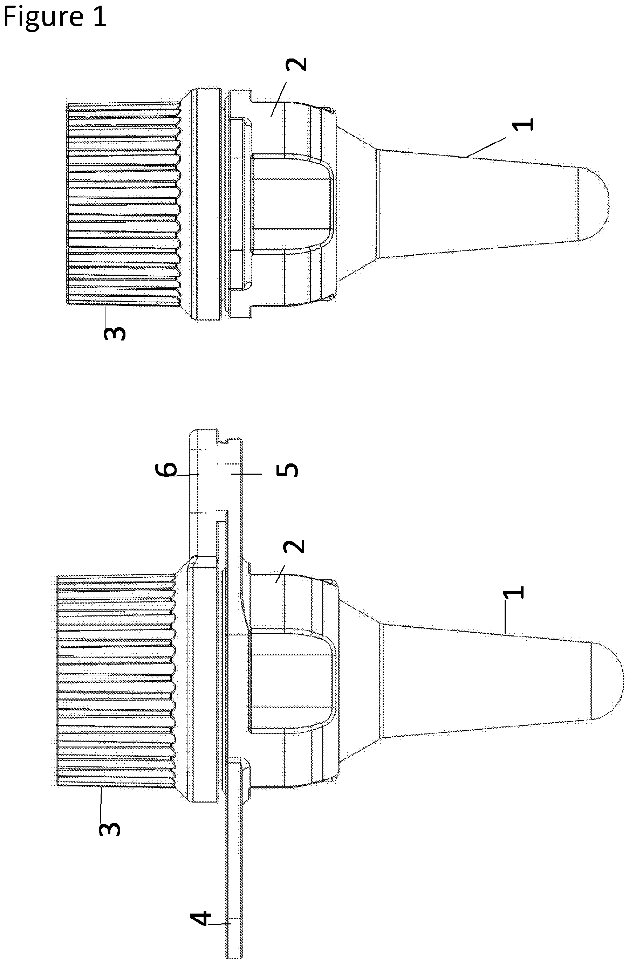

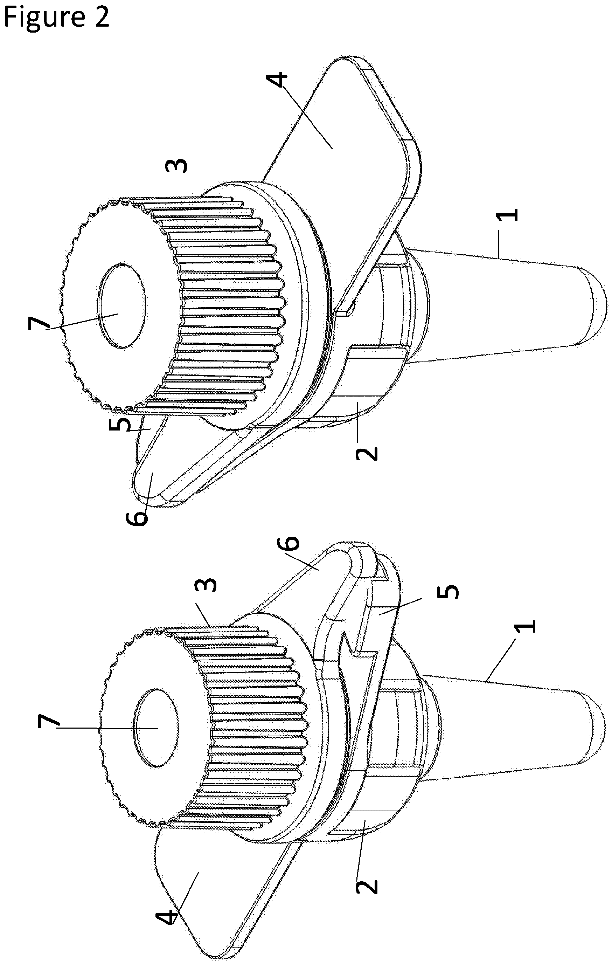

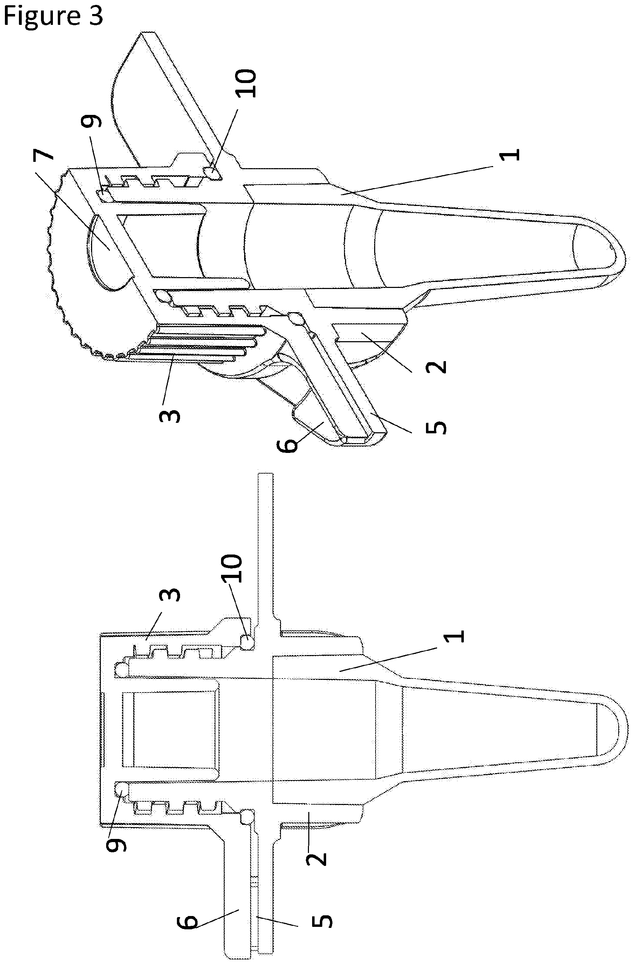

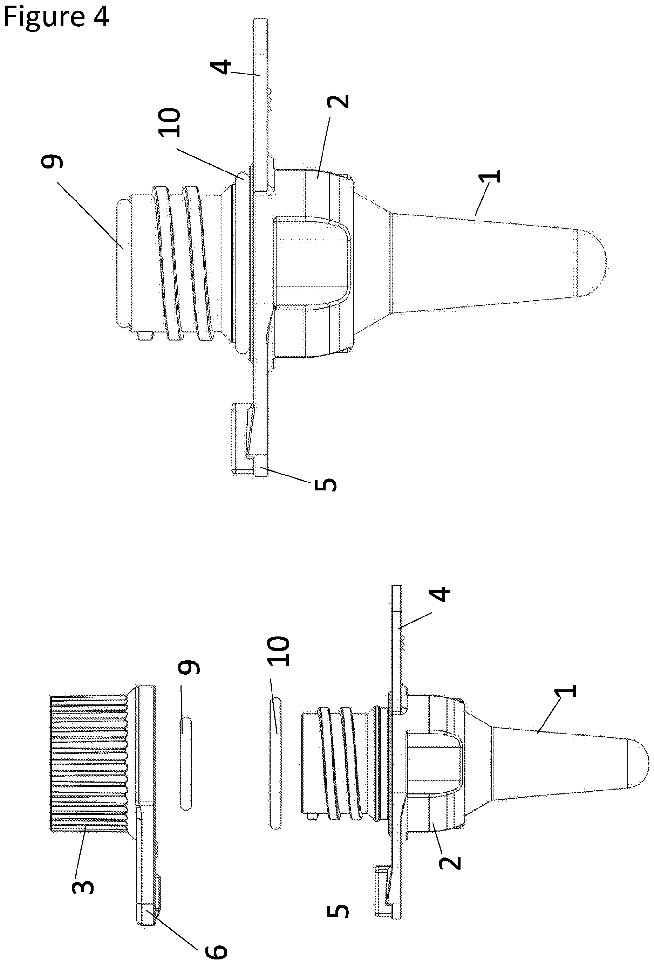

Figure 1-4 show an exemplary vessel of the disclosure for example that can be used in combination with the methods of the disclosure as part of the closed tube, direct from crude sample, detection process. It features a screw capped lid sealed with an o-ring to ensure biosecurity of the vessel. It is formed by a 2 shot injection moulding process whereby the major walls are formed from carbon loaded polypropylene (50-60%) and the minor wall contains a clear polypropylene window on one side. This clear window and the top of the vessel are the first shot and the carbon loaded polymer is the second shot.
1 = reaction vessel chamber made out of the carbon loaded polymer (note how 2 is overmoulded onto the top of 1)
2= 2nd shot injection which contains the screw thread feature and locating means for 2nd 0-ring (10) also the flat surface for holding and writing (4) and the bottom of the lock feature (5)
3= easy to hold cap section, moulded as one part from clear thermosplastic, contains the window (7) and the top part of the lock feature (6)
4= flat "wing I believe you called it" for holding the vessel in gloved hands and carrying barcode/being able to write on
5= bottom of the lock feature
6=top of the lock feature
7= clear/polished window section for taking the optical readings
8= moulded screw thread in (2) the 2nd shot injection
9= smaller o-ring/gasket that goes inside the cap at the top of the screw thread
10= larger gasket that is placed at the base of the screw thread (8) on part 2

The cap is a separate single shot item to which a rubber o-ring has been placed.

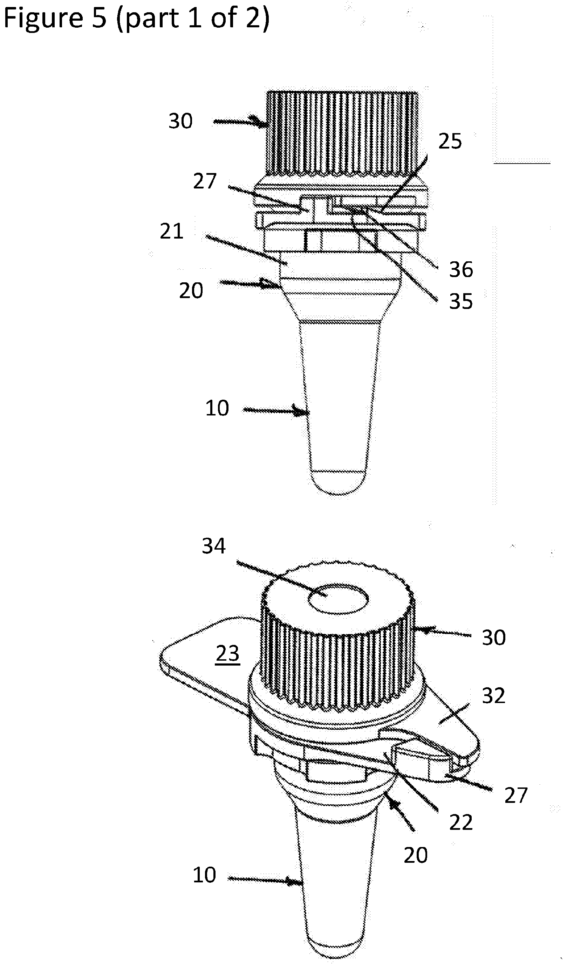

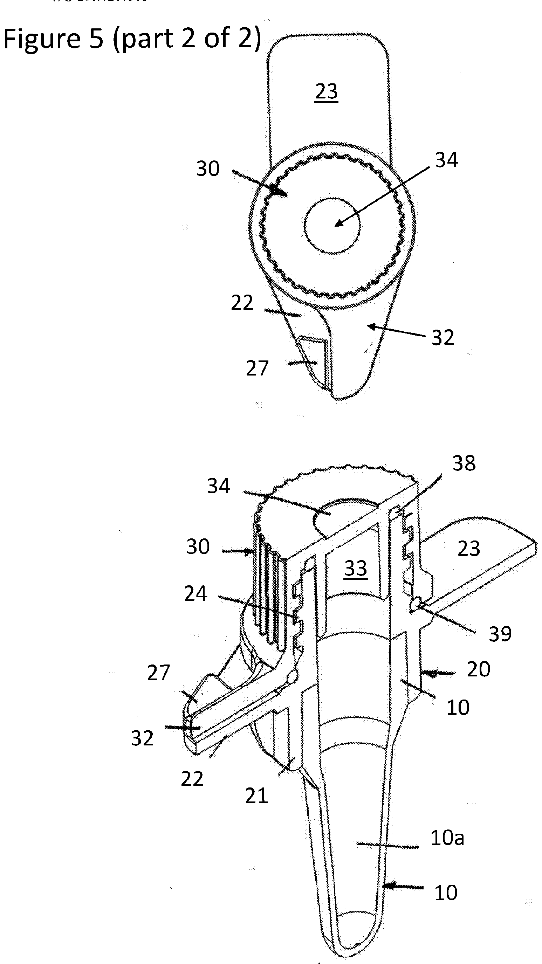

Figure 5 shows further images of the same or alternative embodiment of the vessel according to the disclosure, with the following numbered features.

The reaction vessel shown in the drawings comprises a reaction chamber portion 10, a cap holder portion 20 and a cap 30.

The reaction chamber portion 10 comprises a tapered cylinder 10a of microtitre capacity and a mandrel 10b to which is moulded the cap holder portion 20. The reaction chamber portion 10 is formed of carbon loaded polyurethane.

The cap holder portion 20 comprises a right cylindrical collar portion 21, and a cap attachment portion formed with a lock/stop wing 22, a label wing 23 and a screw threaded manifold 24. The manifold 24 is arranged for three turns of the cap 30 to fasten same.

The lock/stop wing 22 has a ramp 25 followed by a fall step 26, and a stop 27.

The cap holder portion 20 is formed of polyurethane which, being less thermally conductive than the reaction chamber portion 10 restricts heat loss upward from the latter.

The cap 30 comprises a knurled, internally threaded body 31 with a lock wing 32 and an internal reinforcement 33. In the roof of the cap 30 is a window 34. The lock wing 32 has formed thereon a ramp 35 with a step 36. Ramp 35 and step 36 are constructed to cooperate with the ramp 25 and the fall step 26 on the lock/stop wing 22. The lock wing 32 further has an edge 37 arranged to abut the stop 27 when the step 36 abuts the fall step 26. The cap 30 is also formed of polyurethane.

O-ring seals 38 and 39 are provided, for sealing between the cap holder portion 20 and the cap 30 at the upper end of the cap holder portion 20 and the lower end of the manifold respectively.

The reaction vessel is manufactured thus: the reaction chamber portion 10 is injection moulded. The cap holder portion 20 is then moulded on to the mandrel portion 10b. An appropriate O-ring seal 36 is fitted into the cap 30 and a seal 39 on to the cap holder 20 at the base of the screw thread.

In use, the reaction vessel is stood and held in a suitable holder. The sample and reagents are loaded into the reaction vessel. Then the cap 30 is screwed onto the holder until the stops 26 and 36 engage and the edge 37 abuts the stop 27. Then the vessel and contents are ready for further stages in the designated process.

Key features:
Two-shot-required for optical and thermal use
Separate screw lid-biosecure feature
2 o-rings-biosecure feature
Lid click sealed - Self locking lid closure stop to prevent over tightening, and to give visual indication to the user that the lid is correctly installed / sealed.-biosecure feature clear optical window on top of the lid-optimal for optical interrogation Multiple points of failure in order to prevent release of pathogen-vessel tested to 6 atmospheres pressure.-biosecure feature
Carbon impregnated polypropylene wall-high thermal conductivity
Volume 50-220ul
flat optical viewing window,-improved optics
anti-rotation features to hold tube in holder whilst lid is installed,-biosecure feature vessel is handed so can only be placed in instrument correct way round-utility option for top optics by simply moulding clear lids (polished section in the lid mould). - improved optics
Tab for tube patient ID notation options.-sample id
Total height 35mm (lid installed) widest point (rear of proposed ID tab to front of locking feature) 33mm and largest diameter of central area 14.7mm.-compact vessel

### Example 3

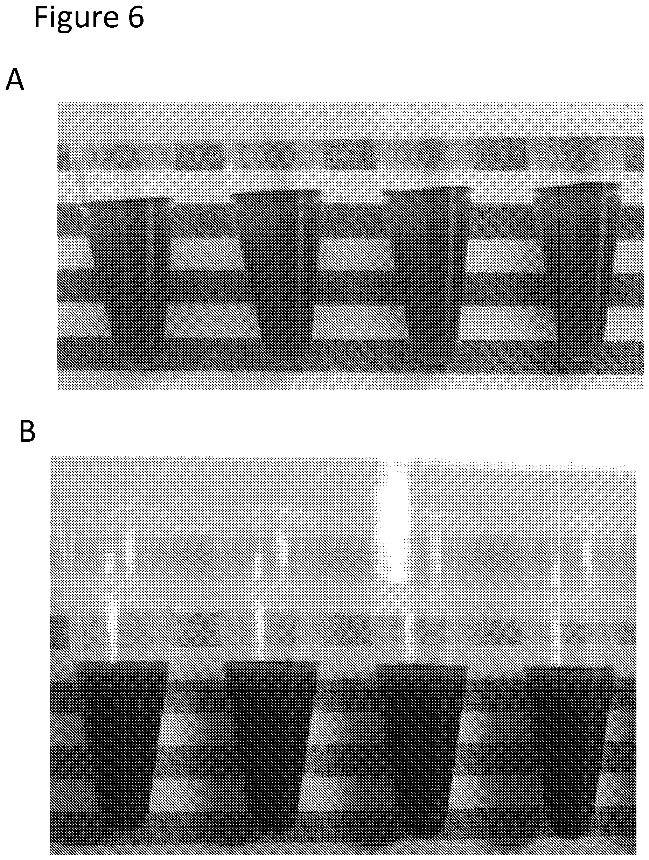

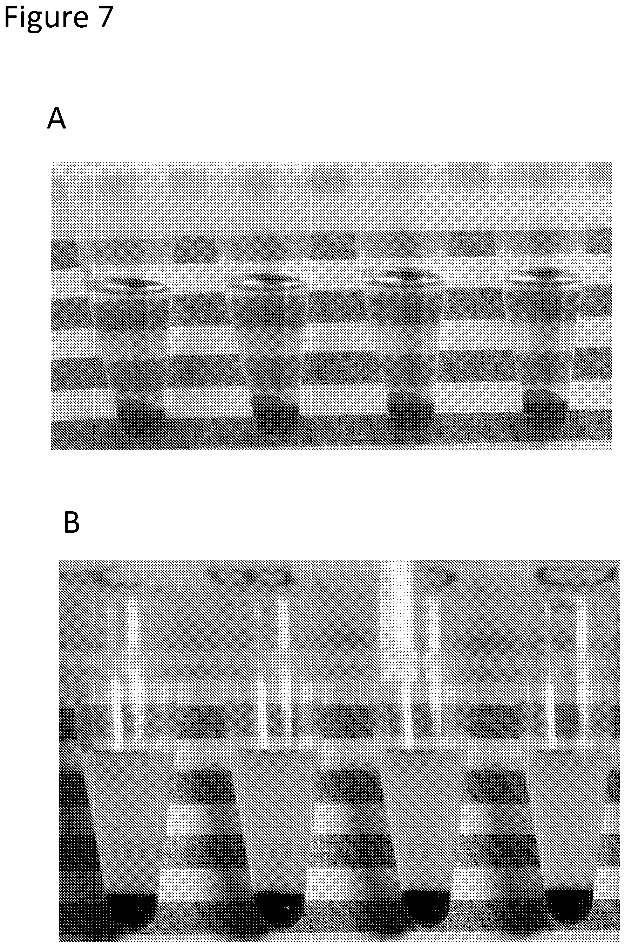

Figures 6 and 7 show tubes containing a PCR reaction with a 15% whole blood sample. It can clearly be seen that where the sample has been centrifuged, the supernatant is much more amenable to the detection of a range of fluorophores.

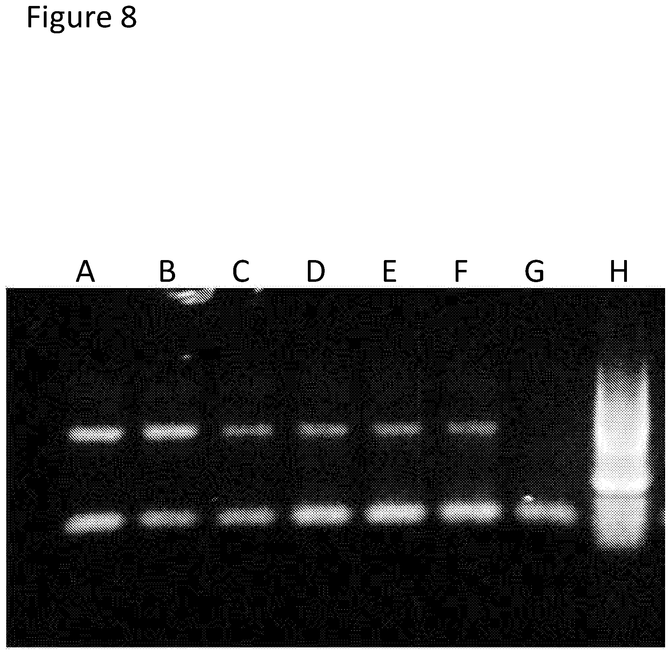

Figure 8 shows the results of a RT-PCR analysis of a range of samples wherein the sample comprises different amounts of blood and wherein the samples have been centrifuged, or have not been centrifuged.

Figure 9 shows the results of a RT-qPCR assay detecting a 106bp amplicon from the Ebola genome, showing that qPCR can be performed when the samples have been centrifuged, but not when the samples have not been centrifuged.

## Claims

1. A method for the detection of a pathogen in a sample that may comprise one or more pathogens, wherein the method comprises performing RT, PCR or RT-PCR and wherein the sample comprises particulate or cellular matter;
**characterised in that** the method comprises:
adding the sample to a vessel that comprises PCR reaction components; and
centrifuging the sample prior to RT and PCR and not during RT or PCR within a closed vessel and wherein the vessel is the same vessel as the PCR is to subsequently be performed in,
and
wherein the centrifugation step is performed:
a) at a speed and duration so as to pellet the particular/cellular material whilst leaving the pathogen in the supernatant; or
b) wherein where the sample is a blood sample, the centrifugation is performed at a speed and for a duration so as to result in pelleting a first fraction of the sample whilst leaving a second fraction of the sample in the supernatant, wherein the first fraction comprises red blood cells and the second fraction comprises white blood cells and any viruses or bacteria present in the sample.

2. The method of claim 1 wherein the sample is a crude sample, optionally:
a) a crude biological sample, optionally a sample taken from an organism, optionally whole blood sample, faeces, urine, cerebral spinal fluid, or eluate from swabs taken from a subject, optionally taken from the eyes, ears, nose or mouth; and/or
b) a crude environmental sample, optionally wherein the sample comprises a food sample, swabs from surfaces or other samples that are not taken directly from an organism.

3. The method of any of claims 1 or 2 wherein the pathogens are selected from:
a) the group consisting of viruses, bacteria, fungi; and/or
b) class 4 or 3 pathogens; and/or pathogens that cause the diseases selected from the group consisting of:
c) Viral haemorrhagic fevers selected from the group consisting of Ebola, Lassa fever, Marburg virus disease, Rift valley fever, Congo fever and yellow fever; and/or
d) Japanese encephalitis, Dengue, Zika, Chikungunya.

4. The method of any of claims 1-3 wherein the sample comprises a substance, or releases a substance, that is inhibitory to PCR, optionally releases a substance upon heating that is inhibitory to PCR.

5. The method of any of claims 1-4 wherein the centrifugation is performed at a speed and for a duration so as to result in pelleting a first fraction of the sample whilst leaving a second fraction of the sample in the supernatant and wherein the sample is blood and the first fraction comprises red blood cells and the second fraction comprises white blood cells and any viruses or bacteria present in the sample.

6. The method of any of claims 1-5 wherein the centrifugation is performed at less than 1000g, optionally between 200g and 1000g, between 300g and 900g, between 400g and 800g, between 500g and 700g, optionally 600g; and wherein the centrifugation is performed for less than 60 seconds, optionally between 5 and 60 seconds, optionally between 10 and 55, 15 and 50, 20 and 45, 25 and 40, 30 and 35 seconds;
optionally wherein the centrifugation is performed at 500g for 30 seconds.

7. The method of any of claims 1-6 wherein the PCR reaction components are lyophilised.

8. The method according to claim any one of claims 1-7 where the PCR is qPCR or the RT-PCR is RT-qPCR, optionally wherein the excitation wavelength used to excite the fluorophore associated with the qPCR is between 630nm-645nm, optionally between 633nm-642nm; and/or the emitted light is collected at a wavelength of between 650nm-750nm,
optionally wherein the fluorophore is excited at a wavelength of around 475nm and/or 635nm; and/or the emitted light is collected at a wavelength of around 520-580nm and 660-750nm.

9. The method according to any of claims 1-8 wherein the sample comprises at least 5% of the PCR reaction volume, optionally at least 6%, 7%, 8%, 9%, 10%, 11%,12%,13%,14%,15%,16%,17%,18%,19%, 20%, 21%, 22%, 23%, 24%, 25%, 26%, 27%, 28%, 29%, 30%, 31%, 32%, 33%, 34% or 35% or greater; optionally comprises 13% of the reaction volume.

10. The method according to any one of claims 1-9 wherein the method comprises detection of the presence of an amplification product.

11. A method for diagnosing a subject as being infected with a target pathogen, wherein the method comprises the method according to any of claims 1-10.

## Patentansprüche

1. Verfahren zur Nachweis eines Pathogens in einer Probe, die gegebenenfalls ein oder mehrere Pathogene enthält, wobei das Verfahren das Durchführen einer RT, PCR oder RT-PCR umfasst und wobei die Probe partikelförmiges oder zelluläres Material umfasst;
**dadurch gekennzeichnet, dass** das Verfahren umfasst:
Zugeben der Probe zu einem Gefäß, das PCR-Reaktionskomponenten umfasst; und
Zentrifugieren der Probe vor der RT und PCR und nicht während der RT oder PCR innerhalb eines geschlossenen Gefäßes und wobei das Gefäß dasselbe Gefäß ist, in dem nachfolgend die PCR durchgeführt wird,
und
wobei der Zentrifugationsschritt:
a) mit einer Geschwindigkeit und für eine Dauer durchgeführt wird, sodass das partikelförmige/zelluläre Material pelletiert wird, während das Pathogen im Überstand zurückbleibt; oder
b) wobei, wenn die Probe eine Blutprobe ist, die Zentrifugation mit einer Geschwindigkeit und für eine Dauer durchgeführt wird, die in der Pelletierung einer ersten Fraktion der Probe resultieren, während eine zweite Fraktion der Probe im Überstand zurückbleibt, wobei die erste Fraktion rote Blutkörperchen umfasst und die zweite Fraktion weiße Blutkörperchen und jegliche in der Probe vorhandenen Viren oder Bakterien umfasst.

2. Verfahren nach Anspruch 1, wobei die Probe eine rohe Probe ist, gegebenenfalls:
a) eine rohe biologische Probe, gegebenenfalls eine Probe, die von einem Organismus entnommen wurde, gegebenenfalls eine Vollblutprobe, Stuhl, Urin, Cerebrospinalflüssigkeit oder ein Eluat von Abstrichen, die von einem Individuum genommen wurden, gegebenenfalls von den Augen, den Ohren, der Nase oder dem Mund; und/oder
b) eine rohe Umweltprobe, wobei die Probe gegebenenfalls eine Lebensmittelprobe, Abstriche von Oberflächen oder andere Proben umfasst, die nicht direkt von einem Organismus entnommen wurden.

3. Verfahren nach einem der Ansprüche 1 oder 2, wobei die Pathogene ausgewählt sind aus:
a) der Gruppe von Viren, Bakterien, Pilzen; und/oder
b) Pathogenen der Klasse 4 oder 3; und/oder Pathogenen, die Erkrankungen verursachen, die aus folgender Gruppe ausgewählt sind:
c) viralen hämorrhagischen Fiebern, die aus der aus Ebola, Lassa-Fieber, Marburg-Virus-Erkrankungen, Rifttalfieber, Kongofieber und Gelbfieber bestehenden Gruppe ausgewählt sind; und/oder
d) Japanischer Enzephalitis, Dengue, Zika, Chikungunya.

4. Verfahren nach einem der Ansprüche 1-3, wobei die Probe eine Substanz umfasst oder eine Substanz abgibt, die PCR-hemmend wirkt, gegebenenfalls beim Erhitzen eine Substanz abgibt, die PCR-hemmend wirkt.

5. Verfahren nach einem der Ansprüche 1-4, wobei die Zentrifugation mit einer Geschwindigkeit und für eine Dauer durchgeführt wird, die zur Pelletierung einer ersten Fraktion der Probe resultiert, während eine zweite Fraktion der Probe im Überstand zurückbleibt, und wobei die Probe Blut ist und die ersten Fraktion rote Blutkörperchen umfasst und die zweite Fraktion weiße Blutkörperchen und jegliche in der Probe vorhandenen Viren oder Bakterien umfasst.

6. Verfahren nach einem der Ansprüche 1-5, wobei die Zentrifugation mit weniger als 1000 g, gegebenenfalls mit zwischen 200 g und 1000 g, zwischen 300 g und 900 g, zwischen 400 g und 800 g, zwischen 500 g und 700 g, gegebenenfalls mit 600 g durchgeführt wird; und wobei die Zentrifugation weniger als 60 Sekunden lang, gegebenenfalls zwischen 5 und 60 Sekunden lang, gegebenenfalls zwischen 10 und 55, 15 und 50, 20 und 45, 25 und 40, 30 und 35 Sekunden lang durchgeführt wird;
wobei die Zentrifugation gegebenenfalls 30 Sekunden lang mit 500 g durchgeführt wird.

7. Verfahren nach einem der Ansprüche 1-6, wobei die PCR-Reaktionskomponenten gefriergetrocknet sind.

8. Verfahren nach einem der Ansprüche 1-7, wobei die PCR eine qPCR oder die RT-PCR eine RT-qPCR ist, wobei die Anregungswellenlänge, die zum Anregen des der qPCR zugeordneten Fluorophors zwischen 630 nm - 645 nm, gegebenenfalls zwischen 633 nm - 642 nm beträgt; und/oder das emittierte Licht bei einer Wellenlänge zwischen 650 nm - 750 nm aufgefangen wird,
wobei das Fluorophor gegebenenfalls mit einer Wellenlänge von etwa 475 nm und/oder 635 nm angeregt wird; und/oder das emittierte Licht bei einer Wellenlänge von etwa 520-580 nm und 660-750 nm aufgefangen wird.

9. Verfahren nach einem der Ansprüche 1-8, wobei die Probe zumindest 5 % des PCR-Reaktionsvolumens, gegebenenfalls zumindest 6 %, 7 %, 8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 % oder 35 % oder mehr umfasst; gegebenenfalls 13 % des Reaktionsvolumens umfasst.

10. Verfahren nach einem der Ansprüche 1-9, wobei das Verfahren den Nachweis des Vorhandenseins eines Amplifikationsprodukts umfasst.

11. Verfahren zur Diagnose eines Individuums als mit einem Zielpathogen infiziert, wobei das Verfahren ein Verfahren nach einem der Ansprüche 1-10 umfasst.

## Revendications

1. Procédé de détection d'un pathogène dans un échantillon qui peut comprendre un ou plusieurs pathogènes, dans lequel le procédé comprend la réalisation d'une RT, d'une PCR ou d'une RT-PCR et dans lequel l'échantillon comprend une matière particulaire ou cellulaire ;
**caractérisé en ce que** le procédé comprend :
l'ajout de l'échantillon dans un récipient qui comprend des composants de réaction de PCR ; et
la centrifugation de l'échantillon avant une RT et une PCR et pas au cours d'une RT ou d'une PCR à l'intérieur d'un récipient fermé et dans lequel le récipient est le même récipient que celui dans lequel la PCR doit être ultérieurement réalisée, et
dans lequel l'étape de centrifugation est réalisée :
a) à une vitesse et pendant une durée permettant de granuler la matière particulaire ou cellulaire tout en laissant le pathogène dans le surnageant ; ou
b) dans lequel l'échantillon est un échantillon sanguin, la centrifugation est réalisée à une vitesse et pendant une durée permettant de granuler une première fraction de l'échantillon tout en laissant une seconde fraction de l'échantillon dans le surnageant, dans lequel la première fraction comprend des globules rouges et la seconde fraction comprend des globules blancs et d'éventuels virus ou bactéries présents dans l'échantillon.

2. Procédé selon la revendication 1 dans lequel l'échantillon est un échantillon brut, facultativement :
a) un échantillon biologique brut, facultativement un échantillon prélevé depuis un organisme, facultativement un échantillon de sang total, des fèces, de l'urine, du fluide cérébrospinal ou un éluat provenant d'écouvillons prélevés depuis un sujet, facultativement prélevés depuis les yeux, les oreilles, le nez ou la bouche ; et/ou
b) un échantillon environnemental brut, facultativement dans lequel l'échantillon comprend un échantillon alimentaire, des écouvillons prélevés sur des surfaces ou d'autres échantillons qui ne sont pas prélevés directement depuis un organisme.

3. Procédé selon la revendication 1 ou 2 dans lequel les pathogènes sont choisis parmi :
a) le groupe constitué de virus, de bactéries, de champignons ; et/ou
b) des pathogènes de classe 4 ou 3 ; et/ou des pathogènes qui provoquent les maladies choisies dans le groupe constitué de :
c) fièvres hémorragiques virales choisies dans le groupe constitué de Ebola, fièvre de Lassa, maladie à virus de Marburg, fièvre de la vallée du Rift, fièvre du Congo et fièvre jaune ; et/ou
d) encéphalite japonaise, Dengue, Zika, Chikungunya.

4. Procédé selon l'une quelconque des revendications 1 à 3 dans lequel l'échantillon comprend une substance, ou libère une substance, qui est inhibitrice d'une PCR, facultativement libère une substance par chauffage qui est inhibitrice d'une PCR.

5. Procédé selon l'une quelconque des revendications 1 à 4 dans lequel la centrifugation est réalisée à une vitesse et pendant une durée permettant de granuler une première fraction de l'échantillon tout en laissant une seconde fraction de l'échantillon dans le surnageant et dans lequel l'échantillon est du sang et la première fraction comprend des globules rouges et la seconde fraction comprend des globules blancs et d'éventuels virus ou bactéries présents dans l'échantillon.

6. Procédé selon l'une quelconque des revendications 1 à 5 dans lequel la centrifugation est réalisée à moins de 1000 g, facultativement entre 200 g et 1000 g, entre 300 g et 900 g, entre 400 g et 800 g, entre 500 g et 700 g, facultativement à 600 g ; et dans lequel la centrifugation est réalisée pendant moins de 60 secondes, facultativement entre 5 et 60 secondes, facultativement entre 10 et 55, 15 et 50, 20 et 45, 25 et 40, 30 et 35 secondes ;
facultativement dans lequel la centrifugation est réalisée à 500 g pendant 30 secondes.

7. Procédé selon l'une quelconque des revendications 1 à 6 dans lequel les composants de réaction de PCR sont lyophilisés.

8. Procédé selon l'une quelconque des revendications 1 à 7 dans lequel la PCR est une qPCR ou la RT-PCR est une RT-qPCR, facultativement dans lequel la longueur d'onde d'excitation utilisée pour exciter le fluorophore associé à la qPCR est entre 630 nm et 645 nm, facultativement entre 633 nm et 642 nm ; et/ou la lumière émise est collectée à une longueur d'onde entre 650 nm et 750 nm, facultativement dans lequel le fluorophore est excité à une longueur d'onde d'environ 475 nm et/ou 635 nm ; et/ou la lumière émise est collectée à une longueur d'onde entre environ 520 nm et 580 nm et entre environ 660 nm et 750 nm.

9. Procédé selon l'une quelconque des revendications 1 à 8 dans lequel l'échantillon comprend au moins 5 % du volume de réaction de PCR, facultativement au moins 6 %, 7 %, 8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 % ou 35 % ou plus ; comprend facultativement 13 % du volume de réaction.

10. Procédé selon l'une quelconque des revendications 1 à 9 dans lequel le procédé comprend la détection de la présence d'un produit d'amplification.

11. Procédé de diagnostic qu'un sujet est infecté d'un pathogène cible, dans lequel le procédé comprend le procédé selon l'une quelconque des revendications 1 à 10.
